# EUROPEAN PATENT APPLICATION

(11) **EP 2 537 513 A2**
(43) Date of publication of application: **26.12.2012**
(21) Application number: 11159269.7
(22) Date of filing: 22.03.2011
(51) Int. Cl.: A61K 8/66, A61K 8/97

(54) **Use of anogeissus extract for fibrillin production in skin**

(30) Priority: 11.03.2011 US 451654 P
(71) Applicant: ELC Management LLC, New York, NY 10153 (US)
(72) Inventor: M. Daly, Susan, New York, NY 11725 (US); Declercq, Lieve, B-2180, Ekeren (BE)
(74) Representative: Hirsch & Associés

(57) **Abstract**

Use of at least one extract from the *Anogeissus* genus for treating human skin by producing fibrillin and thereby increasing skin elasticity.

## Description

### Technical Field

The invention is in the field of the use of an extract from a plant from the *Anogeissus* genus topical compositions for application to keratinous surfaces such as skin, hair, or nails in order to produce fibrillin.

### Background of the Invention

Companies that make skin care products are always looking for new and improved ingredients and formulas. It is known that skin cells, or keratinocytes, are vulnerable to daily assaults from the environment such as cigarette smoke, wind, sun, environmental toxins, and so on. Skin is also known to suffer from its own challenges such as dryness, oiliness, and natural degradation with age. Zinc is often applied to skin in combination with other ingredients in order to treat various conditions. For example, it is believed that zinc pyrithione is useful for inhibiting sebum production, thereby reducing the oily appearance of skin and keratin fibers. Further to sebum inhibition activity, zinc PCA is known to act as an anti-inflammatory agent in skin care compositions. Zinc PCA and Zinc pyrithione have also been used for various skin benefits, including maintenance of generally healthy-looking skin and even for wound care ointments.

Various natural ingredients and extracts are also used to deliver skin care benefits. Such ingredients include aloe vera gel, green tea, white birch, grape seed oil, and many others. While each of these ingredients have shown to provide individual benefits to skin, many ingredients may be exotic or otherwise expensive, which creates formulation limitations due to cost. Therefore, there is an ongoing need for a natural skin care active which provides a synergistic benefit when combined with zinc-based ingredients, such that cost-effective benefits may be delivered to skin.

### Summary of the Invention

The invention is directed a personal care composition comprising at least one extract from the *Anogeissus* genus; from about 0.001 to about 5.00% of zinc PCA; a dermatologically acceptable carrier; and methods of treating skin with the composition herein.

### Detailed Description

All percentages mentioned herein are percentages by weight of the complete composition unless otherwise indicated. The compositions of the invention may be further described as set forth herein.

### Anogeissus Extract

*Anogeissus* is a genus of trees which are indigenous to Asia and Africa, belonging to the family *Combretaceae*. Extracts from the *Anogeissus* genus provide improved skin texture, feel, hydration, moisturization, and appearance of lines, wrinkles, uneven pigmentation, mottling, and other age-related or undesirable skin conditions. There are about eight species in the *Anogeissus* genus, including *Anogeissus acumintata, bentii, dhofarica, latifolia, leiocarpus, rotundifolia, schimperi,* and *sericea*. The extracts may come from the leaves, stems, seeds, bark, flowers, roots, and so on. In one embodiment the extract may be an aqueous or aqueous/alcoholic extract of the plant parts. The *Anogeissus* extract may be present in the composition in amounts ranging from about 0.0001-75%, preferably from about 0.0005-50%, and most preferably from about 0.001-10%.

In one embodiment, the compositions comprise an extract from the bark *of Anogeissus Leiocarpus. Anogeissus Leiocarpus* is a tall evergreen tree native to the savannas of Tropical

Africa, and it is the only West African species of the *Anogeissus* species. One form of *Anogeissus Leiocarpus* is a brownish powder sold by Actives International LLC under the trade name ViaPure Anogeissus. By way of contrast, the *Anogeissus latifolia* species is a small to medium-sized tree native to India, Nepal, Myanmar, and Sri Lanka. It is commonly referred to as axlewood and is generally known to contain significant amounts of tannin.

### Zinc

The zinc salt of L-PCA (pyroglutamine acid, PCA) is an active physiological regulator of sebum production especially formulated for oily skin problems. Zinc is generally known to reduce sebum (suet) secretion. Its activity is enhanced by the content of PCA amino acid. It has been surprisingly discovered that that zinc PCA, in combination with plant extracts from the *Anogeissus* genus, uniquely aids in stimulating fibrillin production. Fibrillin is known to provide a network for elastin deposition, which aids in skin elasticity and provides a youthful skin appearance.

It has been found that zinc-based skin care ingredients are particularly useful in combination with *Anogeissus* extracts for skin rejuvenation benefits. A preferred combination includes zinc PCA in combination with the *Anogeissus leiocarpus* species in a skin care formulation. It is believed that *Anogeissus leiocarpus* acts in concert with zinc PCA to provide a synergistic fibrillin production benefit. As previously discussed, it is believed that fibrillin provides a framework for elastin development, and elastin is important for skin resiliency and youthfulness. Zinc PCA is commercially available from Aston Chemicals under the trademark, Zincidone®. Zinc PCA is present at levels from about 0.001 to about 0.5%, alternatively from about 0.01 to about 0.3%, and alternatively from about 0.10 to about 0.2%.

Zinc PCA may also be combined with zinc pyrithione. Zinc pyrithione may be present at levels of from about 0.01 to about 0.5%, alternatively from about 0.10 to about 0.3%, and alternatively from about 0.10 to about 0.2%.

### DNA Repair Enzyme

The compositions may also contain one or more DNA repair enzymes. DNA repair enzymes may be present in an amount from about 0.00001 to about 35%, preferably from about 0.00005 to about 30%, more preferably from about 0.0001 to about 25% of one or more DNA repair enzymes.

DNA repair enzymes as disclosed in U.S. Patent Nos. 5,077,211; 5,190,762; 5,272,079; and 5,296,231, are suitable for use in the compositions and method of the invention. One example of such a DNA repair enzyme may be purchased from AGI/Dermatics under the trade name Roxisomes®, and has the INCI name *Arabidopsis Thaliana* extract. It may be present alone or in admixture with lecithin and water. This DNA repair enzyme is known to be effective in repairing 8-oxo-diGuanine base mutation damage.

Another type of DNA repair enzyme that may be used is one that is known to be effective in repairing 06-methyl guanine base mutation damage. It is sold by AGI/Dermatics under the tradename Adasomes®, and has the INCI name Lactobacillus ferment, which may be added to the composition of the invention by itself or in admixture with lecithin and water.

Another type of DNA repair enzyme that may be used is one that is known to be effective in repairing T-T dimers. The enzymes are present in mixtures of biological or botanical materials. Examples of such ingredients are sold by AGI/Dermatics under the tradenames Ultrasomes® or Photosomes®. Ultrasomes® comprises a mixture of *Micrococcus* lysate (an end product of the controlled lysis of various species *of Micrococcus*), lecithin, and water. Photosomes® comprises a mixture of plankton extract (which is the extract of marine biomass which includes one or more of the following organisms: thalassoplankton, green micro-algae, diatoms, greenish-blue and nitrogen-fixing seaweed), water, and lecithin.

Another type of DNA repair enzyme may be a component of various inactivated bacterial lysates such as *Bifida* lysate or *Bifida* ferment lysate, the latter a lysate from *Bifido* bacteria which contains the metabolic products and cytoplasmic fractions when *Bifido* bacteria are cultured, inactivated and then disintegrated. This material has the INCI name *Bifida* Ferment Lysate.

### Other Ingredients

The composition may be in the form of an aqueous solution, gel, or suspension; or in the form of an emulsion - either water in oil or oil in water. The composition may also be anhydrous. The composition may be in the liquid, semi-solid, or solid form.

If present as an aqueous solution or dispersion, the amount of water present may range from about 0.01-99%, and the amount of dissolved or dispersed solids from about 10 to 99.99%, in addition to the DNA repair enzyme and *Anogeissus* extract mentioned about in the amounts set forth.

If the composition of the invention is in the emulsion form, it may comprise from about 0.1-99% water and from about 0.1-80% oil in addition to DNA repair enzyme and *Anogeissus* extract as specified herein and in the amounts set forth.

If the composition of the invention is in an anhydrous form, it may contain from about 0.1-99% oil in addition to the *Anogeissus* extract and DNA repair enzymes and in the amounts set forth herein.

### Humectants

The composition may contain one or more humectants. If present, they may range from about 0.1 to 75%, preferably from about 0.5 to 70%, more preferably from about 0.5 to 40%. Examples of suitable humectants include glycols, sugars, and the like. Suitable glycols are in monomeric or polymeric form and include polyethylene and polypropylene glycols such as PEG 4-10, which are polyethylene glycols having from 4 to 10 repeating ethylene oxide units; as well as C₁₋₆ alkylene glycols such as propylene glycol, butylene glycol, pentylene glycol, and the like. Suitable sugars, some of which are also polyhydric alcohols, are also suitable humectants. Examples of such sugars include glucose, fructose, honey, hydrogenated honey, inositol, maltose, mannitol, maltitol, sorbitol, sucrose, xylitol, xylose, and so on. Also suitable is urea. Preferably, the humectants used in the composition of the invention are C₁₋₆, preferably C₂₋₄ alkylene glycols, most particularly butylene glycol, glycerin, propylene glycol, or hexylene glycol.

### Botanical Extracts

It may be desirable to incorporate one more botanical extracts into the composition in addition to the *Anogeissus* extract. If present suggested ranges are from about 0.000 to 20%, preferably from about 0.0005 to 15%, more preferably from about 0.001 to 10%. Suitable botanical extracts include extracts from plants (herbs, roots, flowers, fruits, seeds) such as flowers, fruits, vegetables, and so on, including yeast ferment extract, *Padiha Pavonica* extract, *Therms Thermophilis* ferment extract, *Camelina Sativa* seed oil, *Boswellia Serrata* extract, olive extract, *Acacia Dealbata* extract, *Acer Saccharinum* (sugar maple), *Acidopholus, Acorus, Aesculus, Agaricus, Agave, Agrimonia,* algae, aloe, citrus, *Brassica,* cinnamon, orange, apple, blueberry, cranberry, peach, pear, lemon, lime, pea, seaweed, caffeine, green tea, chamomile, willowbark, mulberry, poppy, and those set forth on pages 1646 through 1660 of the CTFA Cosmetic Ingredient Handbook, Eighth Edition, Volume 2. Further specific examples include, but are not limited to, *Glycyrrhiza Glabra, Salix Nigra, Macrocycstis Pyrifera, Pyrus Malus, Saxifraga Sarmentosa, Vitis Vinifera, Morus Nigra, Scutellaria Baicalensis, Anthemis Nobilis, Salvia Sclarea, Prunus Amygdalus, Rosmarinus Officianalis, Sapindus makurossi, Caesalpinia spinosa, Citrus Medica Limonum, Panax Ginseng, Siegesbeckia Orientalis, Mangifera Indicia, Fructus Mume, Psidium Guajava, Ascophyllum Nodosum, Centaurium erythrea, Glycine Soja* extract, *Beta Vulgaris, Haberlea Rhodopensis, Polygonum Cuspidatum, Citrus Aurantium Dulcis, Vitis Vinifera, Selaginella Tamariscina, Humulus Lupulus, Citrus Reticulata* Peel, *Punica Granatum, Asparagopsis, Curcuma Longa, Menyanthes Trifoliata, Helianthus Annuus, Hordeum Vulgare, Cucumis Sativus, Evernia Prunastri, Evernia Furfuracea, Kola Acuminata,* glycyrretinic acid, and mixtures thereof.

### Peptides

It may be desired to incorporate one or more peptides into the composition. The term "peptide" means from 2 to 20 amino acids connected by peptide bonds. If so, suggested ranges are from about 0.001 to 20%, preferably from about 0.005 to 15%, more preferably from about 0.01 to 10%. Preferred are biologically active peptides including those set forth in the C.T.F.A. International Cosmetic Ingredient Dictionary and Handbook, Eleventh Edition, 2006, page 2712. Such peptides include, but are not limited to the CTFA names: Acetyl Hexapeptide-1, 7, 8; Acetyl Pentapeptide-1, 2, 3, or 5; Acetyl Tripeptide-1; Acetyl Dipeptide-1 cetyl ester; Acetyl Glutamyl Heptapeptide-3; Acetyl Glutamyl Hexapeptide-6; Acetyl Monofluoropeptide-1; Heptapeptide-1, 2, or 3; Hexapeptide-1, 2, 3, 4, 5, 6, 7, 8, 9, 10, 11, 12, 13, or 14; Manganese Tripeptide-1; Myristoyl Hexapeptide-5, 12, or 13; Myristoyl Nonapeptide-2; Myristoyl Pentapeptide-4; Myristoyl Tetrapeptide-4 or 6; Myristoyl Tripeptide-4; Nisin, Nonapeptide-1 or 2; Oligopeptide-1, 2, 3, 4, 5, 6, 7, 8, 9 or 10; Palmitoyl Hexapeptide-14; Palmitoyl Pentapeptide-4; Palmitoyl Pentapeptide-4 or 5; Palmitoyl Tripeptide-1 or 5; Pentapeptide-1, 2, 3, 4, 5, or 6; Tetrapeptide-1, 2, 3" 4, 5, 6, or 7; Tripeptide-1, 2, 3, 4, or 5; or Palmitoyl Oligopeptides.

In one preferred embodiment the composition comprises Acetyl Hexapeptide-8, having the trade name Argireline®.

### Oils

The composition may also comprise one or more oils in the form of natural, synthetic, or silicone oils. The term "oil" means that the ingredient is pourable at room temperature, e.g. 25° C. Oils may be volatile or non-volatile. The term "volatile" means that the oil has vapor pressure greater than about 2 mm of mercury at 20° C. The term "non-volatile" means that the oil has a vapor pressure of less than about 2 mm. of mercury at 20° C. If present, suggested ranges are from about 0.1 to 60%, preferably from about 0.5 to 45%.

Examples of volatile oils include volatile linear, cyclic or branched silicones such as cyclopentasiloxane, cyclohexasiloxane (2 cst), hexamethyldisiloxane (0.65 cst, centistokes), octamethyltrisiloxane (1.0 cst), decamethyltetrasiloxane (1.5 cst), or dodecamethylpentasiloxane (2.0 cst); or branched volatile silicones such as methyl trimethicone (1.5 cst). Also suitable are volatile paraffinic hydrocarbons such as isododecane, isohexadecane, C11-14 alkanes, and mixtures thereof.

Non-volatile oils include linear silicones commonly referred to as dimethicone; phenyl substituted silicones such as phenyl dimethicone, phenyl trimethicone, trimethylsiloxy phenyldimethicone, cetyl dimethicone, perfluorodimethicone, phenethyl dimethicone, and the like.

Non-volatile oils may also include esters or hydrocarbons. Esters include C1-10 alkyl esters of C1-20 carboxylic acids. One preferred type of ester is a fatty acid (C6-22) ester of a straight or branched chain saturated or unsaturated C1-22 alkyl. Examples include esters that have a low viscosity, e.g. ranging from 10-100 cst at room temperature. Examples of such esters include but are not limited to jojoba esters.

Other non-volatile oils include sterols such as phytosterols, phytosphingosine, and similar plant sterols.

### Thickeners

Suitable thickeners may be incorporated into the composition. If so, suggested ranges are from about 0.0001-45%, preferably from about 0.0005-40%.

Examples of thickeners include animal, vegetable, mineral, silicone, or synthetic waxes which may have melting points ranging from about 30 to 150° C. including but not limited to Examples of such waxes include waxes made by Fischer-Tropsch synthesis, such as polyethylene or synthetic wax; or various vegetable waxes such as bayberry, candelilla, ozokerite, acacia, beeswax, ceresin, cetyl esters, flower wax, citrus wax, carnauba wax, jojoba wax, japan wax, polyethylene, microcrystalline, rice bran, lanolin wax, mink, montan, bayberry, ouricury, ozokerite, palm kernel wax, paraffin, avocado wax, apple wax, shellac wax, clary wax, spent grain wax, grape wax, and polyalkylene glycol derivatives thereof such as PEG6-20 beeswax, or PEG-12 carnauba wax; or fatty acids or fatty alcohols, including esters thereof, such as hydroxystearic acids (for example 12-hydroxy stearic acid), tristearin, tribehenin, and so on.

Also suitable as thickening agents are silicas, silicates, silica silylate, and alkali metal or alkaline earth metal derivatives thereof. These silicas and silicates are generally found in the particulate form and include silica, silica silylate, magnesium aluminum silicate, and the like.

Silicone elastomers may also be used as thickening agents. Such elastomers include those that are formed by addition reaction-curing, by reacting an SiH-containing diorganosiloxane and an organopolysiloxane having terminal olefinic unsaturation, or an alpha-omega diene hydrocarbon, in the presence of a platinum metal catalyst. Such elastomers may also be formed by other reaction methods such as condensation-curing organopolysiloxane compositions in the presence of an organotin compound via a dehydrogenation reaction between hydroxyl-terminated diorganopolysiloxane and SiH-containing diorganopolysiloxane or alpha omega diene; or by condensation-curing organopolysiloxane compositions in the presence of an organotin compound or a titanate ester using a condensation reaction between an hydroxyl-terminated diorganopolysiloxane and a hydrolysable organosiloxane; peroxide-curing organopolysiloxane compositions which thermally cure in the presence of an organoperoxide catalyst.

One type of elastomer that may be suitable is prepared by addition reaction-curing an organopolysiloxane having at least 2 lower alkenyl groups in each molecule or an alpha-omega diene; and an organopolysiloxane having at least 2 silicon-bonded hydrogen atoms in each molecule; and a platinum-type catalyst. While the lower alkenyl groups such as vinyl, can be present at any position in the molecule, terminal olefinic unsaturation on one or both molecular terminals is preferred. The molecular structure of this component may be straight chain, branched straight chain, cyclic, or a network. These organopolysiloxanes are exemplified by methylvinylsiloxanes, methylvinylsiloxane-dimethylsiloxane copolymers, dimethylvinylsiloxy-terminated dimethylpolysiloxanes, dimethylvinylsiloxy-terminated dimethylsiloxane-methylphenylsiloxane copolymers, dimethylvinylsiloxy-terminated dimethylsiloxane-diphenylsiloxane-methylvinylsiloxane copolymers, trimethylsiloxy-terminated dimethylsiloxane-methylvinylsiloxane copolymers, trimethylsiloxy-terminated dimethylsiloxane-methylphenylsiloxane-methylvinylsiloxane copolymers, dimethylvinylsiloxy-terminated methyl(3,3,3-trifluoropropyl) polysiloxanes, and dimethylvinylsiloxy-terminated dimethylsiloxane-methyl(3,3,-trifluoropropyl)siloxane copolymers, decadiene, octadiene, heptadiene, hexadiene, pentadiene, or tetradiene, or tridiene.

Curing proceeds by the addition reaction of the silicon-bonded hydrogen atoms in the dimethyl methylhydrogen siloxane, with the siloxane or alpha-omega diene under catalysis using the catalyst mentioned herein. To form a highly crosslinked structure, the methyl hydrogen siloxane must contain at least 2 silicon-bonded hydrogen atoms in each molecule in order to optimize function as a crosslinker.

The catalyst used in the addition reaction of silicon-bonded hydrogen atoms and alkenyl groups, and is concretely exemplified by chloroplatinic acid, possibly dissolved in an alcohol or ketone and this solution optionally aged, chloroplatinic acid-olefin complexes, chloroplatinic acid-alkenylsiloxane complexes, chloroplatinic acid-diketone complexes, platinum black, and carrier-supported platinum.

Examples of suitable silicone elastomers for use in the compositions of the invention may be in the powder form, or dispersed or solubilized in solvents such as volatile or non-volatile silicones, or silicone compatible vehicles such as paraffinic hydrocarbons or esters. Examples of silicone elastomer powders include vinyl dimethicone/methicone silesquioxane crosspolymers like Shin-Etsu's KSP-100, KSP-101, KSP-102, KSP-103, KSP-104, KSP-105, hybrid silicone powders that contain a fluoroalkyl group like Shin-Etsu's KSP-200 which is a fluoro-silicone elastomer, and hybrid silicone powders that contain a phenyl group such as Shin-Etsu's KSP-300, which is a phenyl substituted silicone elastomer; and Dow Coming's DC 9506. Examples of silicone elastomer powders dispersed in a silicone compatible vehicle include dimethicone/vinyl dimethicone crosspolymers supplied by a variety of suppliers including Dow Coming Corporation under the tradenames 9040 or 9041, GE Silicones under the tradename SFE 839, or Shin-Etsu Silicones under the trade names KSG-15, 16, 18. KSG-15 has the CTFA name cyclopentasiloxane/dimethicone/vinyl dimethicone crosspolymer. KSG-18 has the INCI name phenyl trimethicone/dimethicone/phenyl vinyl dimethicone crossoplymer. Silicone elastomers may also be purchased from Grant Industries under the Gransil trademark. Also suitable are silicone elastomers having long chain alkyl substitutions such as lauryl dimethicone/vinyl dimethicone crosspolymers supplied by Shin Etsu under the tradenames KSG-31, KSG-32, KSG-41, KSG-42, KSG-43, and KSG-44. Cross-linked organopolysiloxane elastomers useful in the present invention and processes for making them are further described in U.S. Pat. No. 4,970,252 to Sakuta et al., issued Nov. 13, 1990; U.S. Pat. No. 5,760,116 to Kilgour et al., issued Jun. 2, 1998; U.S. Pat. No. 5,654,362 to Schulz, Jr. et al. issued Aug. 5, 1997; and Japanese Patent Application JP 61-18708, assigned to Pola Kasei Kogyo KK.

Polysaccharides may be suitable aqueous phase thickening agents. Examples of such polysaccharides include naturally derived materials such as agar, agarose, alicaligenes polysaccharides, algin, alginic acid, acacia gum, amylopectin, chitin, dextran, cassia gum, cellulose gum, gelatin, gellan gum, hyaluronic acid, hydroxyethyl cellulose, methyl cellulose, ethyl cellulose, pectin, sclerotium gum, xanthan gum, pectin, trehelose, gelatin, and so on.

Also suitable are different types of synthetic polymeric thickeners. One type includes acrylic polymeric thickeners comprised of monomers A and B wherein A is selected from the group consisting of acrylic acid, methacrylic acid, and mixtures thereof; and B is selected from the group consisting of a C₁₋₂₂ alkyl acrylate, a C₁₋₂₂ alky methacrylate, and mixtures thereof are suitable. Acrylic polymer solutions include those sold by Seppic, Inc., under the tradename Sepigel® or those sold under the tradename Aristoflex®.

Also suitable are acrylic polymeric thickeners that are copolymer of A, B, and C monomers wherein A and B are as defined above, and C has the general formula: 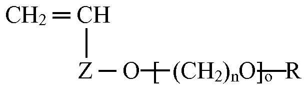 wherein Z is -(CH₂)ₘ; wherein m is 1-10, n is 2-3, o is 2-200, and R is a C₁₀₋₃₀ straight or branched chain alkyl. Examples of the secondary thickening agent above, are copolymers where A and B are defined as above, and C is CO, and wherein n, o, and R are as above defined. Examples of such secondary thickening agents include acrylates/steareth-20 methacrylate copolymer, which is sold by Rohm & Haas under the tradename Acrysol ICS-1.

Also suitable are acrylate based anionic amphiphilic polymers containing at least one hydrophilic unit and at least one allyl ether unit containing a fatty chain. Preferred are those where the hydrophilic unit contains an ethylenically unsaturated anionic monomer, more specificially a vinyl carboxylic acid such as acrylic acid, methacrylic acid or mixtures thereof, and where the allyl ether unit containing a fatty chain corresponds to the monomer of formula:

CH₂ = CR'CH₂OBₙR

in which R' denotes H or CH₃, B denotes the ethylenoxy radical, n is zero or an integer ranging from 1 to 100, R denotes a hydrocarbon radical selected from alkyl, arylalkyl, aryl, alkylaryl and cycloalkyl radicals which contain from 8 to 30 carbon atoms, preferably from 10 to 24, and even more particularly from 12 to 18 carbon atoms. More preferred in this case is where R' denotes H, n is equal to 10 and R denotes a stearyl (C18) radical. Anionic amphiphilic polymers of this type are described and prepared in U.S. Patent Nos. 4,677,152 and 4,702,844. Among these anionic amphiphilic polymers, polymers formed of 20 to 60% by weight acrylic acid and/or methacrylic acid, of 5 to 60% by weight lower alkyl methacrylates, of 2 to 50% by weight allyl ether containing a fatty chain as mentioned above, and of 0 to 1% by weight of a crosslinking agent which is a well-known copolymerizable polyethylenic unsaturated monomer, for instance diallyl phthalate, allyl (meth)acrylate, divinylbenzene, (poly)ethylene glycol dimethacrylate and methylenebisacrylamide. One commercial example of such polymers are crosslinked terpolymers of methacrylic acid, of ethyl acrylate, of polyethylene glycol (having 10 EO units) ether of stearyl alcohol or steareth-10, in particular those sold by the company Allied Colloids under the names ***SALCARE*** SC80 and ***SALCARE*** SC90, which are aqueous emulsions containing 30% of a crosslinked terpolymer of methacrylic acid, of ethyl acrylate and of steareth-10 allyl ether (40/50/10).

Also suitable are acrylate copolymers such as Polyacrylate-3 which is a copolymer of methacrylic acid, methylmethacrylate, methylstyrene isopropylisocyanate, and PEG-40 behenate monomers; Polyacrylate-10 which is a copolymer of sodium acryloyldimethyltaurate, sodium acrylate, acrylamide and vinyl pyrrolidone monomers; or Polyacrylate-11, which is a copolymer of sodium acryloyldimethylacryloyldimethyl taurate, sodium acrylate, hydroxyethyl acrylate, lauryl acrylate, butyl acrylate, and acrylamide monomers.

Also suitable are crosslinked acrylate based polymers where one or more of the acrylic groups may have substituted long chain alkyl (such as 6-40, 10-30, and the like) groups, for example acrylates/C₁₀-₃₀ alkyl acrylate crosspolymer which is a copolymer of C 10-30 alkyl acrylate and one or more monomers of acrylic acid, methacrylic acid, or one of their simple esters crosslinked with the allyl ether of sucrose or the allyl ether of pentaerythritol. Such polymers are commonly sold under the Carbopol or Pemulen tradenames and have the CTFA name carbomer.

One particularly suitable type of aqueous phase thickening agent are acrylate based polymeric thickeners sold by Clariant under the Aristoflex trademark such as Aristoflex AVC, which is ammonium acryloyldimethyltaurate/VP copolymer; Aristoflex AVL which is the same polymer has found in AVC dispersed in mixture containing caprylic/capric triglyceride, trilaureth-4, and polyglyceryl-2 sesquiisostearate; or Aristoflex HMB which is ammonium acryloyldimethyltaurate/beheneth-25 methacrylate crosspolymer, and the like.

Also suitable as thickening agents are various polyethylene glycols (PEG) derivatives where the degree of polymerization ranges from 1,000 to 200,000. Such ingredients are indicated by the designation "PEG" followed by the degree of polymerization in thousands, such as PEG-45M, which means PEG having 45,000 repeating ethylene oxide units. Examples of suitable PEG derivatives include PEG 2M, 5M, 7M, 9M, 14M, 20M, 23M, 25M, 45M, 65M, 90M, 115M, 160M, 180M, and the like.

Also suitable are polyglycerins which are repeating glycerin moieties where the number of repeating moieties ranges from 15 to 200, preferably from about 20-100. Examples of suitable polyglycerins include those having the CTFA names polyglycerin-20, polyglycerin-40, and the like.

### Surfactants

If desired, the compositions of the invention may contain one or more surfactants. This is particularly desirable when the composition is in the form of an aqueous gel or emulsion. If present, the surfactant may range from about 0.001 to 50%, preferably from about 0.005 to 40%, more preferably from about 0.01 to 35% by weight of the total composition. Suitable surfactants may be silicone or organic, nonionic, anionic, amphoteric or zwitterionic. Such surfactants include, but are not limited to, those set forth herein and are well known in the art.

### Vitamins and Antioxidants

The compositions may comprise from about 0.0001% to about 50%, alternatively from about 0.001% to about 10%, alternatively from about 0.01% to about 5%, and alternatively from about 0.1% to about 1%, of one or more vitamins. Herein, "vitamins" means vitamins, pro-vitamins, and their salts, isomers and derivatives. Non-limiting examples of suitable vitamins include: vitamin B compounds (including B1 compounds, B2 compounds, B3 compounds such as niacinamide, niacinnicotinic acid, tocopheryl nicotinate, C1-C18 nicotinic acid esters, and nicotinyl alcohol; B5 compounds, such as panthenol or "pro-B5", pantothenic acid, pantothenyl; B6 compounds, such as pyroxidine, pyridoxal, pyridoxamine; carnitine, thiamine, riboflavin); vitamin A compounds, and all natural and/or synthetic analogs of Vitamin A, including retinoid compounds such as retinol, retinyl acetate, retinyl palmitate, retinoic acid, retinaldehyde, retinyl propionate; carotenoids (pro-vitamin A); vitamin D compounds; vitamin K compounds; vitamin E compounds, or tocopherol, including tocopherol sorbate, tocopherol acetate, other esters of tocopherol and tocopheryl compounds; vitamin C compounds, including ascorbate, ascorbyl esters of fatty acids, and ascorbic acid derivatives, for example, ascorbyl phosphates such as magnesium ascorbyl phosphate and sodium ascorbyl phosphate, ascorbyl glucoside, and ascorbyl sorbate; and vitamin F compounds, such as saturated and/or unsaturated fatty acids. In one embodiment, the composition comprises a vitamin selected from the group consisting of vitamin B compounds, vitamin C compounds, vitamin E compounds and mixtures thereof. Alternatively, the vitamin is selected from the group consisting of niacinamide, tocopheryl nicotinate, pyroxidine, panthenol, vitamin E, vitamin E acetate, ascorbyl phosphates, ascorbyl glucoside, and mixtures thereof.

### Parabens

It is further recognized that there is an ongoing need for stable cosmetic systems which maximize the use of natural ingredients and therefore avoid negative side effects of certain synthetic preservatives. Accordingly, in one embodiment, the compositions are substantially free of parabens. Known parabens include butylparaben, ethylparaben, methylparaben, propylparaben, and mixtures thereof. The term, "substantially free" as used herein, means that less than 0.050%, and preferably no paraben materials are present in the composition.

### Dermatologically Acceptable Carrier

The compositions comprise a dermatologically acceptable carrier for the skin care active materials. "Dermatologically acceptable," as used herein, means that the compositions or components described are suitable for use in contact with human keratinous tissue without undue toxicity, incompatibility, instability, allergic response, and the like. The compositions may comprise from about 50% to about 99.99%, alternatively from about 60% to about 99.9%, alternatively from about 70% to about 98%, and alternatively from about 80% to about 95% of the composition.

The carrier can be a wide variety of types, non-limiting examples of which include solutions, dispersions, emulsions and combinations thereof. Herein, "emulsions" generally contain an aqueous phase and an oil phase. The oils may be derived from animals, plants, or petroleum, may be natural or synthetic, and may include silicone oils. Emulsion carriers include, but are not limited to oil-in-water, water-in-oil and water-in-oil-in-water emulsions. In one embodiment, the carrier comprises an oil-in-water emulsion, a water-in-oil emulsion a silicone-in-water emulsion, and/or a water-in-silicone emulsion. The emulsions may comprise from about 0.01% to about 10%, and alternatively from about 0.1% to about 5%, of a nonionic, anionic or cationic emulsifier, and combinations thereof. Suitable emulsifiers are disclosed in, for example, U.S. Pat. No. 3,755,560, U.S. Pat. No. 4,421,769, and McCutcheon's Detergents and Emulsifiers, North American Edition, pages 317-324 (1986).

The invention will be further described in connection with the following example which is set forth for purposes of illustration only.

### EXAMPLES

A skin treatment serum is prepared as follows:

| Ingredient | A | B | c | D | E |
|---|---|---|---|---|---|
| Water | QS100 | QS100 | QS100 | QS100 | QS100 |
| Butylene glycol | 5.20 | 5.00 | 5.20 | 5.30 | 5.00 |
| Dimethicone | 4.25 | 2.00 | 2.80 | 3.25 | 4.25 |
| Acetyl hexapeptide-8 | 2.00 | 2.00 | 2.00 | 2.00 | 2.00 |
| Arginine/salicylic acid/tocopheryl acetate/mixed soy phospholipids | 2.00 | 2.00 | 2.00 | 2.00 | 2.00 |
| Jojoba esters | 1.50 | 1.50 | 1.50 | 1.50 | 1.50 |
| Glycerin | 1.25 | 1.25 | 1.25 | 1.25 | 1.25 |
| Dicaprylyl carbonate | 1.00 | 1.00 | 1.00 | 1.00 | 1.00 |
| Methyl trimethicone | 1.00 | 1.00 | 1.00 | 1.00 | 1.00 |
| Dimethicone/Polysilicone-11 | 1.00 | 1.00 | 1.00 | 1.00 | 1.00 |
| Water/*Prunus amygdalus* ducis (sweet almond) seed extract | 1.00 | 1.00 | 1.00 | 1.00 | 1.00 |
| Silica | 0.75 | 0.75 | 0.75 | 0.75 | 0.75 |
| Carbo mer | 0.50 | 0.50 | 0.50 | 0.50 | 0.50 |
| Ammonium acryloyldimethyltaurate/VP copolymer | 0.50 | 0.50 | 0.50 | 0.50 | 0.50 |
| Glycerin/water/Sapindus mukurossi fruit extract/Caesalpinia spinosa gum | 0.50 | 0.50 | 0.50 | 0.50 | 0.50 |
| Butylene glycol/Centaurium erythraea (Centaury) extract | 0.50 | 0.50 | 0.50 | 0.50 | 0.50 |
| Siegesbeckia Orientalis extract/glycerin | 0.50 | 0.50 | 0.50 | 0.50 | 0.50 |
| Tromethamine | 0.45 | 0.45 | 0.45 | 0.45 | 0.45 |
| Phenoxyethano 1 | 0.43 | 0.43 | 0.43 | 0.43 | 0.43 |
| Caprylyl glycol/phenoxyethanol/hexylene glycol | 0.40 | 0.40 | 0.40 | 0.40 | 0.40 |
| Caffeine | 0.20 | 0.20 | 0.20 | 0.20 | 0.20 |
| Water/butylenes glycol/Scutellaria baicalensis (root) extract/Pyrus Malus (Apple) extract/Cucumis Sativus (cucumber) extract | 0.20 | 0.20 | 0.20 | 0.20 | 0.20 |
| Tetrahexyldecyl ascorbate | 0.20 | 0.20 | 0.20 | 0.20 | 0.20 |
| Phytosphingosine | 0.20 | 0.20 | 0.20 | 0.20 | 0.20 |
| *Anogeissus Leiocarpus* extract | 0.15 | 0.0015 | 5.00 | 0.50 | 0.75 |
| Fragrance | 0.15 | 0.15 | 0.15 | 0.15 | 0.15 |
| Glycyrretinic acid | 0.10 | 0.10 | 0.10 | 0.10 | 0.10 |
| *Mahgifera Indicia* (Mango) leaf extract | 0.10 | 0.10 | 0.10 | 0.10 | 0.10 |
| *Psidium Guajava* (Guava) fruit extract | 0.10 | 0.10 | 0.10 | 0.10 | 0.10 |
| *Zinc PCA* | 0.10 | 0.20 | 0.10 | 0.10 | 0.10 |
| *Zinc Pyrithione* | 0.10 | 0.00 | 0.00 | 0.20 | 0.10 |
| *Polygonum cuspidatum* root extract | 0.05 | 0.05 | 0.05 | 0.05 | 0.05 |
| Sodium hyaluronate | 0.02 | 0.02 | 0.02 | 0.02 | 0.02 |
| Phosphatidyl choline | 0.009 | 0.009 | 0.009 | 0.009 | 0.009 |
| Sodium chloride | 0.009 | 0.009 | 0.009 | 0.009 | 0.009 |
| Phytosterol | 0.0019 | 0.0019 | 0.0019 | 0.0019 | 0.0019 |
| Disodium phosphate | 0.001 | 0.001 | 0.001 | 0.001 | 0.001 |
| Ursolic acid/Rosmarinus Officinalis (Rosemary) extract | 0.001 | 0.001 | 0.001 | 0.001 | 0.001 |
| Anthemis Nobilis (Chamomile) flower extract | 0.0005 | 0.0005 | 0.0005 | 0.0005 | 0.0005 |
| Micrococcus Luteus powder | 0.0005 | 0.0005 | 0.0005 | 0.0005 | 0.0005 |
| Potassium phosphate monobasic | 0.0002 | 0.0002 | 0.0002 | 0.0002 | 0.0002 |
| Potassium chloride | 0.0002 | 0.0002 | 0.0002 | 0.0002 | 0.0002 |
| Phosphatidyl ethanolamine | 0.00009 | 0.00009 | 0.00009 | 0.00009 | 0.00009 |
| Disodium EDTA | 0.00006 | 0.00006 | 0.00006 | 0.00006 | 0.00006 |
| BHT | 0.00002 | 0.00002 | 0.00002 | 0.00002 | 0.00002 |
| Oleic acid | 0.00002 | 0.00002 | 0.00002 | 0.00002 | 0.00002 |
| Sodium hydroxide | 0.000007 | 0.000007 | 0.000007 | 0.000007 | 0.000007 |
| OGGI fermentate | 0.000002 | 0.000002 | 0.000002 | 0.000002 | 0.000002 |

The composition is prepared by combining the ingredients and mixing well.

While the invention has been described in connection with the preferred embodiment, it is not intended to limit the scope of the invention to the particular form set forth but, on the contrary, it is intended to cover such alternatives, modifications, and equivalents as may be included within the spirit and scope of the invention as defined by the appended claims.

## Claims

1. Use of at least one extract from the *Anogeissus* genus, and preferably the *Anogeissus leiocarpus* species of said *Anogeissus* genus, to produce fibrillin in human skin.

2. A use according to claim 1, wherein said extract is formulated in a personal care composition to provide a benefit selected from improved skin texture, feel, hydration, moisturization; preventing the appearance of lines, wrinkles, uneven pigmentation, mottling, and age-related skin conditions.

3. A use according to claim 2, wherein said personal care composition comprises at least one additional skin care active, said active being preferably selected from a DNA repair enzyme, a peptide, a botanical extract, and zinc PCA.

4. A use according to claim 3, wherein said DNA repair enzyme is selected from one or more of OGGI, or found in *Micrococcus* lysate, *Bifidus, Arabidopsis Thaliana* extract, *Lactobacillus,* plankton extract, or mixtures thereof or ferments thereof;

5. A use according to claims 3 or 4, wherein the peptide is selected from a hexapeptide, pentapeptide, tripeptide, dipeptide, a palmitoyl derivative, or mixtures thereof.

6. A use according to claim 5, wherein said peptide is Acetyl Hexapeptide-8.

7. A use according to any one of claims 3-6, wherein said botanical extract is selected from *Scutellaria baicalensis, Pyrus malus, Cucumis sativus,* glycyrretinic acid, *Mangifera indicia, Psidium guava, Polygonum cuspidatum, Rosmarinus officinalis, Anthemis nobilis,* caffeine, and mixtures thereof.
